# EUROPEAN PATENT APPLICATION

(11) **EP 2 556 793 A1**
(43) Date of publication of application: **13.02.2013**
(21) Application number: 12180210.2
(22) Date of filing: 13.08.2012
(51) Int. Cl.: A61B 5/00, A61B 5/0402, A61B 5/0404

(54) **System, method, mobile equipment and software for first aid treatment**

(30) Priority: 12.08.2011 FI 20115795
(71) Applicant: Satucon Oy, 70210 Kuopio (FI)
(72) Inventor: Jäntti, Helena, FI-70620 Kuopio (FI)
(74) Representative: Pitkänen, Hannu Alpo Antero

(57) **Abstract**

The invention relates to a system for first aid treatment, which includes at least one mobile equipment, by which first aid treatment is arranged to be requested, a telephone service on duty, such as an emergency response centre, provided with means and software for receiving and evaluating first aid treatment requests and for activating the system for first aid treatment, into connection with the mobile equipment are arranged means for performing an electrocardiography (ECG) measurement and for sending measurement data to the emergency response centre, and to the emergency response centre are arranged means for analysing the measurement data. In the system according to the invention, at the emergency response centre, there is at least a semi-automatic defibrillator software for determining if the patient is lifeless or alive, based on which information the emergency response centre (12) is arranged to respond to said request for first aid treatment and to notify the user of the mobile equipment on this information. Furthermore, the invention relates to mobile equipment, software and method for activating the system for first aid treatment and for determining if the patient is lifeless or alive.

## Description

The invention relates to a system for first aid treatment, which includes at least one mobile equipment, by which first aid treatment is arranged to be requested, a telephone service on duty, such as an emergency response centre, provided with means and software for receiving and evaluating first aid treatment requests and for activating the system for first aid treatment, into connection with the mobile equipment are arranged means for performing an electrocardiography (ECG) measurement and for transmitting measurement data to the emergency response centre, and to the emergency response centre are arranged means for analysing the measurement data.

Furthermore, the invention relates to mobile equipment, software and method for activating the system for first aid treatment at the emergency response centre.

Death by heart failure is the most common cause of death for the inhabitants of Western Countries. Half of deaths are sudden, leading to resuscitation. In the Western World, there occur resuscitations outside the hospital for about 50 per 100,000 inhabitants per year. For instance in Finland, this means about 3,000 resuscitations a year, in the area of the OECD countries, about half a million resuscitations a year (population 1.19 billion).

Outside the hospital, the person being the eyewitness and caller for help is most often a layperson and he/she calls for help by dialling the number of an emergency response centre (ECR) (in Finland, emergency number 112). The emergency call is almost always dialled by mobile equipment i.e. a mobile phone. The task of a duty officer at the ECR is to determine as soon as possible what has happened and where in order to be able to send assistance suitable for the case at the scene in question. A situation, in which the patient's heart has stopped, is hard to identify for both the layperson and the ECR duty officer. According to various studies, the ECR duty officer identifies about half of lifelessnesses and half are considered to be some other occurrences.

When the heart stops, the patient's possibilities for survival are primarily dependent on time. The prognosis worsens by about 10% for each minute spent lifeless. That is why each minute of time to be saved from the stopping of the patient's heart to the starting of basic life support and the arrival of paramedics to treat the patient is vital.

Basic life support can sustain some kind of blood circulation and the patient's possibilities for survival are improved. The layperson starts the basic life support according to instructions given by the ECR duty officer, but only if lifelessness is identified at the emergency response centre. It has been studied that identifying lifelessness at the emergency response centre improves the patient's prognosis in two ways: the basic life support given by the layperson starts earlier and the system for first aid treatment is activated quickly and effectively, and two ambulances will depart sirens wailing to the scene for resuscitation.

Recently, the identification of lifelessness at the emergency response centre is based on the eyewitness's verbal description of the incident. When being unresponsive and not breathing or having abnormal breathing, the patient is considered lifeless. With these criteria, lifelessness is identified in about half of the cases. The key question for the patient's survival is if the patient seeming lifeless is alive or lifeless already during the emergency call and identifying the patient's situation as soon as possible.

When paramedics face the patient, they first analyse by a defibrillator (e.g. Zoll) the electric rhythm of the patient's heart. Checking the rhythm ensures lifelessness and is the start in the resuscitation charts. According to their level of training, the paramedics use either a semi- or fully-automatic or manual defibrillator. In the manual model, the paramedics must identify the rhythm by themselves but, in the semi- and fully-automatic models, the machine analyses the rhythm and recommends when necessary administering electricity to stop the ventricular fibrillation or ventricular tachycardia in the heart. In different situations, the paramedics also take an electrocardiogram of alive patients with the same device which analyses the rhythm in resuscitation. The electrocardiogram is transmitted for analysing and receiving treatment instructions from the defibrillator to the mobile phone (a PDF file) and e-mail of an emergency physician. However, the arrival of paramedics takes time which is extremely important for the patient's survival.

There exist means connected to mobile equipment for performing an ECG measurement and for transmitting measurement data to the emergency response centre and the emergency response centre is provided with means for analysing the measurement data, but these will not yield information on the patient being alive or lifeless.

The object of this invention is to provide a way more effective than earlier to call out and receive appropriate assistance in connection with a request for first aid treatment and to receive a system for first aid treatment more effectively. A particular object of the invention is to introduce a system, mobile equipment, software and method by means of which it is possible to discover as quickly as possible if a patient seeming lifeless is alive or lifeless.

The characteristic features of the system according to the invention are described in claim 1 enclosed. Furthermore, the invention relates to mobile equipment, software and method for activating the system for first aid treatment the characteristic features of which are depicted in claims 3, 5 and 6.

In the system and method according to the invention, an ECG measurement is performed by mobile equipment by an eyewitness of a cardiac arrest already during an emergency call. At an emergency response centre, measurement data are analysed and, based on it, the system for first aid treatment is activated. When identifying the patient's lifelessness, it is possible to send appropriate assistance for the person having had a cardiac arrest more quickly and, thus, to substantially improve the patient's possibilities for survival. Furthermore when lifelessness is identified correctly at the emergency response centre, the basic life support can be started earlier and for the correct patients by the maker of the emergency call (a layperson) according to the instructions given by the emergency response centre.

In practice, the course of events is the following: A person experiences a heart failure and a layperson notices the incidence and the possible lifelessness of the person. The layperson calls the emergency number and, at the same time, an ECG reading mode is activated in his/her mobile equipment. An ECR duty officer asks the layperson to put the mobile equipment on the chest of the patient seeming lifeless, whereby the mobile equipment measures the patient's ECG and transmits it automatically to the emergency response centre where the ECR duty officer's computer includes software of at least a semi-automatic defibrillator which determines the fact and automatically analyses the ECG obtained to the classes of alive - lifeless. The ECR duty officer passes the information in question to the caller and instructs based on this analysis the layperson to either resuscitate the patient or only to put the patient on his/her side.

According to the embodiment described in the invention, the ECG reader mode can be activated mainly automatically when it is identified e.g. that the equipment is calling the emergency number (in Finland, 112). Then, the ECG reader is immediately ready for the patient's ECG registering. When calling the emergency number or during the emergency call, it is possible along with the activation of the ECG reader to activate a data transmission connection for transmitting the measurement data created by the ECG reader to the emergency response centre. This mode can also be mainly automatic, whereby the caller has no need to activate it separately. Other characteristic features of the system and method according to the invention will be evident from the enclosed claims and more attainable advantages will be listed elsewhere in the description.

Next, the invention, which is not limited to the following embodiments, will be described in more detail with reference to the attached figures in which
Fig. 1 shows an example of a system according to the invention as a schematic figure and
Fig. 2 shows an example of a method according to the invention as a block diagram.

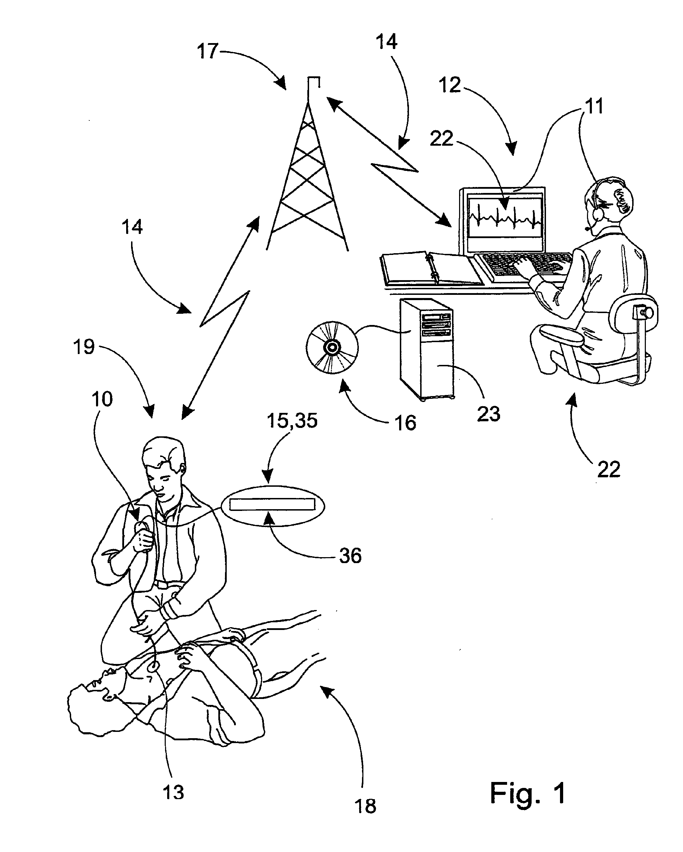

Fig. 1 shows an embodiment of activating a system for first aid treatment according to the invention after a cardiac arrest has been identified on mobile equipment 10 and the whole software. The system includes as parties at least one mobile equipment 10 and a telephone service on duty, such as e.g. an emergency response centre 12. The mobile equipment 10 can be known as such of its basic functions which can be used for requesting first aid treatment by phoning a set emergency number. Equivalently, the emergency response centre 12 is provided with means 11, 23 known as such for receiving and analysing requests for first aid treatment. Normally, this means communications equipment 11 and a computer apparatus 23 with means known as such. The mobile equipment 10 and the emergency response centre 12 can communicate via a data transmission network 17 of known type as such, in which typically, the mobile equipment 10 contacts the emergency response centre 12 for performing a request for first aid treatment.

It is particular of the mobile equipment 10 that into connection with it are arranged means 13, 15 for performing an ECG measurement and for transmitting measurement data 14 via the data transmission network 17 to the emergency response centre 12. The mobile equipment can then include required electrodes which can be e.g. under a removable film. Furthermore, the mobile equipment includes an amplifier for amplifying the ECG signal. Naturally, the mobile equipment 10 can also be such to which an ECG reader can be connected as an external device or even as a sole electrode connectable to the equipment 10 itself by wire or wirelessly. The means 15 of the mobile equipment 10 for transmitting measurement data 14 to the data transmission network 17 can be e.g. data transmission means and properties known as such which enable circuit- or packet-switched data transmission in existing wireless data transmission networks 17 and ones being under development. The transmission of measurement data 14 to the emergency response centre 12 can occur e.g. by a packet data connection which connection is automatically established in connection with the emergency call.

Equivalently, to the emergency response centre 12 are arranged means 11, 23 for receiving measurement data 14 transmitted by the mobile equipment 10 from the data transmission network 17 and for analysing it e.g. by programmatic means 16. An example of the analysing software 16 is semi-automatic Zoll software 16 run by a computer 11. It is also possible to use other, semi-automatic or fully-automatic analysing software suitable for the purpose. The received data 14 can by analysed by the software in question and, based on the analysis, the emergency response centre 12 can respond to the request for first aid treatment by giving information for the person having called for help and by sending assistance to the scene which is determined by the patient's 18 ECG 22. That is, the result of the software 16 is if the patient 18 is lifeless or alive.

According to an embodiment, the means 15 for processing a signal produced by the ECG reader 13 for the mobile equipment 10 to a form suitable for transmitting and for transmitting measurement data 14 by the mobile equipment 10 to the emergency response centre 12 are programmatic. For this, the equipment 10 includes software 15 for producing measurement data 14 from a signal created by the ECG reader 13 and for transmitting the measurement data 14 e.g. in connection with an emergency call to the emergency response centre 12.

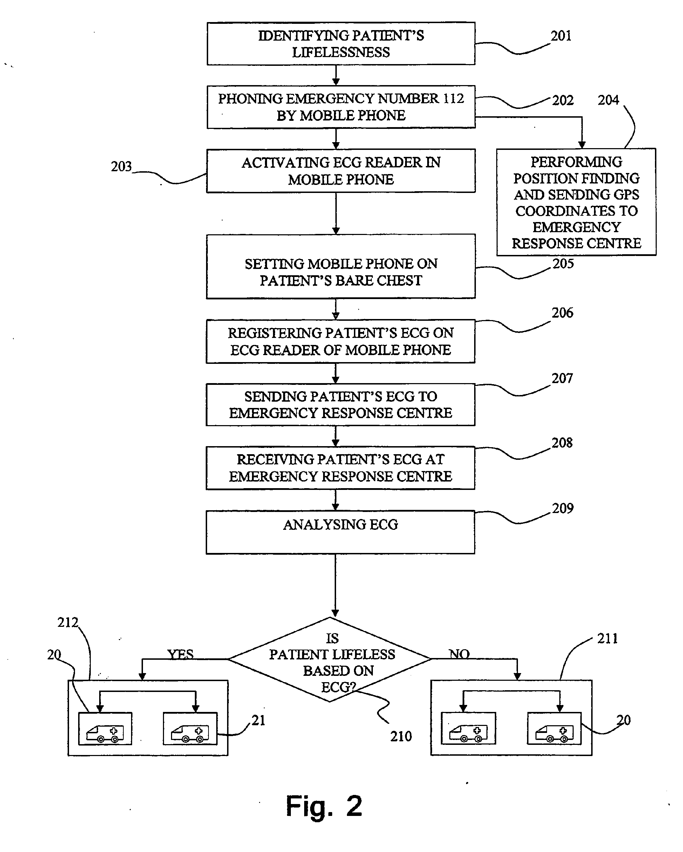

Fig. 2 shows an example of a method for arranging first aid treatment as a block diagram. In connection with the embodiment, we also refer to Fig. 1. As step 201, a helper 19 observes that a patient 18 is lifeless. As a result of the observation in step 202, the helper 19 requests first aid treatment for the patient 18 from an emergency response centre 12 by phoning an emergency number by mobile equipment 10. When the connection to the emergency response centre 12 has been established or already during the attempt of establishing the connection, it is possible as step 203 to activate an ECG reader 13 arranged into connection with the equipment 10 e.g. automatically. At the same time as the connection was established, it is possible as step 204 to perform an automatic positioning update by a GPS position finder arranged into connection with the equipment 10 and to transmit GPS coordinates obtained as its result to the emergency response centre 12. This speeds up the ambulances finding the scene and thus receiving professional assistance.

To implement step 203, the equipment 10 can include software 15 already arranged by the manufacturer or the network operator or arrangeable afterwards, by means of which the functions fundamental for the invention are provided in the equipment 10. The software 15 comprises a storage media 35, which is now the memory of the equipment 10, and a software code 36 written on the storage media 35. The software code 36 is arranged activatable when connecting the equipment 10 to the emergency response centre 12 and to activate the operation of the ECG reader 13 arranged into connection with the equipment 10 and the transmitting of measurement data 14 created by it to the emergency response centre 12.

When the mobile equipment 10 indicates to the helper 19 that an ECG measurement is possible, as step 205, the helper 19 sets his/her mobile equipment 10 on the patient's 18 bare chest. That is, when requesting first aid treatment for the patient 18 the ECG measurement is also performed. When necessary, an ECR duty officer 22 can instruct the helper 19 in setting the mobile equipment 10 on the patient's 18 chest in order to perform the measurement correctly.

As step 206, the measurement is performed i.e. the cardiac rhythm i.e. ECG of the lifeless patient 18 is registered by the ECG reader 13 of the mobile phone 10 at the scene. In the ECG measurement, it is possible to store measurement data 14 e.g. for a set time period. When enough electrocardiogram data 14 have been obtained on the equipment 10 (the equipment 10 and the software 15 arranged to it can identify the sufficiency of the data 14 spontaneously), they are transmitted as step 207 by the mobile phone 10 via the data transmission network 17 to the emergency response centre 12. That is, the transmission of the measurement data 14 to the emergency response centre 12 can also be activated automatically when connecting to the emergency response centre 12 and/or during the connection. As step 208, the data 14 are received at the emergency response centre 12 and stored for analysis. As step 209, the measurement data 14 measured from the patient 18 are analysed at the emergency response centre 12 e.g. by analysis software 16 run by a computer 11. The ECR duty officer's 22 computer 11 includes the computer software 16 to analyse the ECG (e.g. Zoll).

The analysis software 16 gives as the result of analysing the ECG data 14 a notification on the patient 18 being lifeless or alive based on the ECG measurement performed. Utilising this information, the emergency response centre 12 notifies the user of the mobile equipment on this information. Furthermore, the emergency response centre responds to the request for first aid treatment by sending the patient 18 first aid treatment based on the request. That is, based on the analysis of the measurement data 14 the patient 18 is provided with set first aid treatment. If the patient 18 is unresponsive but alive based on the ECG measurement, the ECR duty officer 22 responds to the request for first aid treatment by sending in step 211 one ambulance 20 provided with basic paramedical equipment to the scene. According to the multistage initial care system, this can also be called the basic level or treatment level of emergency care. However, if the patient 18 is lifeless based on the ECG measurement, the ECR duty officer 22 responds to the request for first aid treatment by sending two emergency units 20, 21 to the scene, one of which is e.g. an ambulance capable of basic paramedics and the other a treatment-level ambulance 21 capable of higher-level treatment. The ambulance 21 can also be a first-response unit and the ambulance 20 a basic-level or treatment-level unit. The invention helps to indentify more quickly and reliably the patient's 18 lifelessness (cardiac arrest) and the ECR duty officer 22 is able to send the assistance 20, 21 required to the scene. That is, required assistance is received at the scene considerably more quickly than earlier.

## Claims

1. A system for first aid treatment, which includes
- at least one mobile equipment (10) which includes means for making an emergency call,
- a telephone service on duty (12), such as an emergency response centre (ECR), provided with means (11, 23) and software for receiving and evaluating requests for first aid treatment and for activating the system for first aid treatment,
- into connection with the mobile equipment (10) are arranged means (13, 15) for performing an ECG measurement and for transmitting measurement data (14) to the emergency response centre (12),
- to the emergency response centre (12) are arranged means (16, 23) for analysing the measurement data (14),
***characterised* in that**
- at the emergency response centre, there is at least a semi-automatic defibrillator software for determining if the patient is lifeless or alive, based on which information the emergency response centre (12) is arranged to respond to said request for first aid treatment and to notify the user of the mobile equipment on this information.

2. A system according to claim 1, ***characterised* in that** the means (15) for transmitting measurement data (14) to the emergency response centre (12) are programmatic.

3. Mobile equipment, which is provided with data transmission properties for performing communication in a data transmission network (17), ***characterised* in that** into connection with the mobile equipment (10) are arranged means (13, 15) for performing an ECG measurement and for transmitting measurement data (14) to a set party (12), such as an emergency response centre, to the data transmission network (17),
***characterised* in that** the means (13, 15) for performing the ECG measurement and for receiving a reply message are arranged activatable automatically when connecting to the emergency response centre (12).

4. Software, which comprises a storage media (35) and a software code (36) written on the storage media (35), ***characterised* in that** the software code (36) is arranged activatable when connecting equipment (10) to an emergency response centre (12) and to activate the operation of an ECG reader (13) arranged into connection with the equipment (10).

5. A method for arranging a system for first aid treatment, in which
- an emergency call is made by mobile equipment (10) for activating the system for first aid treatment for a patient (18) from an emergency response centre (12),
- the emergency response centre (12) responds to said request by sending the patient (10 first aid treatment based on the request,
- in connection with the emergency call, an ECG measurement is performed for the cardiac arrest patient (18) the measurement data (14) of which are transmitted by the mobile equipment (10) to the emergency response centre (12),
***characterised* by**
- analysing the measurement data (14) at the emergency response centre (12) by the software of at least a semi-automatic defibrillator to determine if the patient is lifeless or alive,
- activating based on the analysis of the measurement data (14) the system for first aid treatment for the cardiac arrest patient (18), responding to said request for first aid treatment and notifying the user of the mobile equipment on this information.

6. A method according to claim 5, ***characterised* by** activating the ECG measurement when connecting the mobile equipment (10) to the emergency response centre (12) and/or during the connection.

7. A method according to claim 5 or 6, ***characterised* by** activating the transmission of the measurement data (14) to the emergency response centre (12) when connecting the mobile equipment (10) to the emergency response centre (12) and/or during the connection.

8. A method according to any one of claims 5-7, ***characterised* in that,** in the ECG measurement, measurement data (14) are stored for a set period of time, which data (14) are then transmitted to the emergency response centre (12).
